(19) Europäisches Patentamt
European Patent Office
Office européen des brevets

(11) EP 1 345 573 B1

(12) # FASCICULE DE BREVET EUROPEEN

(45) Date de publication et mention de la délivrance du brevet:
**03.12.2008 Bulletin 2008/49**

(21) Numéro de dépôt: **01272063.7**

(22) Date de dépôt: **14.12.2001**

(51) Int Cl.:
***A61K 8/04*** *(2006.01)* ***A61K 8/898*** *(2006.01)*
***A61K 8/81*** *(2006.01)* ***A61Q 5/06*** *(2006.01)*

(86) Numéro de dépôt international:
**PCT/FR2001/004001**

(87) Numéro de publication internationale:
**WO 2002/051364 (04.07.2002 Gazette 2002/27)**

(54) **DISPOSITIF AEROSOL CONTENANT UN POLYURETHANNE ET/OU POLYUREE ET UN POLYMERE FIXANT**

AEROSOLVORRICHTUNG MIT EINEM POLYURETHAN UND/ODER EINEM POLYHARNSTOFF UND EINEM FIXIERPOLYMER

AEROSOL DEVICE CONTAINING A POLYURETHANE AND/OR A POLYUREA AND A FIXING POLYMER

(84) Etats contractants désignés:
**AT BE CH CY DE DK ES FI FR GB GR IE IT LI LU MC NL PT SE TR**

(30) Priorité: **22.12.2000 FR 0016950**

(43) Date de publication de la demande:
**24.09.2003 Bulletin 2003/39**

(73) Titulaire: **L'ORÉAL**
**75008 Paris (FR)**

(72) Inventeurs:
- **PATAUT, Françoise**
  **F-75017 Paris (FR)**
- **BREMENSON, Jean-Luc**
  **F-75019 Paris (FR)**
- **NOCERINO, Cécile**
  **F-75006 Paris (FR)**

(74) Mandataire: **Bourdeau, Françoise et al**
**L'OREAL - D.I.P.I.**
**25-29 Quai Aulagnier**
**92600 Asnières (FR)**

(56) Documents cités:
**EP-A- 0 745 373** **WO-A-00/12056**
**WO-A-97/47535** **FR-A- 2 782 635**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 345 573 B1

## Description

L'invention a pour objet des dispositifs aérosols comprenant un récipient qui contient, dans un milieu cosmétiquement acceptable, un polymère multiséquencé comprenant au moins un motif polyuréthanne et/ou polyurée et au moins un polymère fixant dont la température de transition vitreuse est supérieure ou égale à 40 °C, ces dispositifs étant appropriés pour obtenir un débit initial en composition aérosol supérieur ou égal à 0,55 gramme par seconde. Elle vise également un procédé pour la mise en forme ou le maintien de la coiffure comprenant la mise en oeuvre de ces dispositifs ainsi que leur utilisation pour la fabrication d'une laque ou d'un spray aérosol.

La fixation de la coiffure est un élément important du coiffage qui consiste à maintenir la mise en forme déjà réalisée ou à mettre en forme les cheveux et à les fixer simultanément. Dans ce domaine, on recherche une fixation durable et fiable de la coiffure, tout en évitant de figer la coiffure.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution peut être conditionnée par exemple dans un récipient aérosol approprié mis sous pression à l'aide d'un propulseur.

Le conditionnement sous forme aérosol est spécialement pratique pour l'utilisateur qui obtient sans difficulté une répartition assez homogène du produit. Toutefois, ce type de conditionnement présente l'inconvénient de donner lieu à un dégagement de composés organiques volatiles (VOC) nocifs pour l'environnement. Ils proviennent notamment de la quantité de solvant organique mis en oeuvre et du gaz propulseur choisi pour fabriquer la composition. On porte donc un intérêt tout particulier à la réalisation de compositions cosmétiques conditionnées sous forme aérosol pour lesquelles la quantité de composés organiques volatiles rejetés est plus faible.

Il est connu par le brevet DE 198 07 908 de préparer des compositions de coiffage comprenant un polymère à motif polyuréthanne en tant que polymère fixant. Les dispositifs peuvent toutefois être améliorés en ce qui concerne notamment les propriétés cosmétiques qu'ils confèrent aux cheveux tout en offrant une très bonne qualité de fixation. Ces dispositifs peuvent également être améliorés en ce qui concerne le démêlage des cheveux après l'application des compositions qu'ils contiennent.

De manière surprenante et inattendue, la Demanderesse a découvert, contre toute attente, qu'il était possible de réaliser des dispositifs aérosols qui satisfont aux exigences exprimées ci-dessus, en opérant une sélection d'une part, sur la composition cosmétique et d'autre part, sur les moyens de distribution de cette composition.

L'invention a pour objet un dispositif aérosol comprenant un récipient contenant une composition capillaire formée par un jus et au moins un propulseur ainsi que des moyens de distribution de la composition, caractérisé par le fait que:

(1) la composition comprend, dans un milieu cosmétiquement acceptable :

(i) entre 4,5 et 20 % en poids par rapport au poids total de la composition d'au moins un polycondensat comprenant au moins une séquence polyuréthanne et/ou polyurée;
(ii) entre 0,1 et 5 % en poids par rapport au poids total de la composition d'au moins un polymère fixant, différent(s) du ou des polycondensat(s) (i), dont la température de transition vitreuse est comprise entre 40 et 200 °C ;

(2) le dispositif est approprié pour fournir un débit initial en composition aérosol supérieur ou égal à 0,55 gramme par seconde et inférieur ou égal à 2 grammes par seconde,

le polycondensat présentant un motif répétitif de base repondant à la formule (II')

$$-X'-P-X'-CO-NH-R-NH-CO- \qquad (II')$$

dans laquelle:

- P est un segment polysiloxanique,
- X' représente O et/ou NH, et
- R est un radical divalent choisi parmi les radicaux alkylènes de type aromatique, aliphatique en $C_1$ à $C_{20}$, cycloaliphatique en $C_1$ à $C_{20}$, ces radicaux étant substitués ou non.

Un autre objet de l'invention concerne un procédé pour la mise en forme ou le maintien de la coiffure comprenant la mise en oeuvre de ce dispositif aérosol.

Encore un autre objet de l'invention concerne l'utilisation de ce dispositif pour la fabrication d'une laque ou d'un spray aérosol.

Conformément à la présente invention, la température de transition vitreuse est mesurée par DSC (Differential Scanning Calorimetry). Par température de transition vitreuse (Tg), on entend, selon la présente invention, la Tg du matériau fixant dans l'extrait sec, l'extrait sec étant constitué par l'ensemble des matériaux non volatiles dans le jus, ou matière sèche. La température de transition vitreuse est mesurée à conformément au protocole décrit dans les demandes de brevet EP 845 257 et EP 848 941, déposés par la Demanderesse, à une température de 25°C ± 1°C et à une humidité relative de 46 % ± 2 %.

Les polycondensats comprenant au moins une séquence polyuréthanne et/ou polyurée particulièrement visés par la présente invention sont ceux décrits dans les brevets EP 0 751 162. EP 0 637 600, FR 2 743 297, EP 0 648 485 et EP 814 764 dont la Demanderesse est Titulaire, ainsi que le brevets EP 0 656 021 ou WO 94/03510 de la Société BASF et EP 0 619 111 de la Société National Starch.

Les polycondensats utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

Avantageusement, le segment polysiloxanique P répond à la formule générale ci-après:

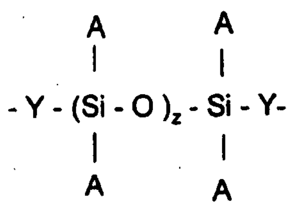

(III)

dans laquelle:

- les radicaux A, qui peuvent être identiques ou différents, sont choisis parmi d'une part les radicaux hydrocarbonées monovalents en $C_1$ à $C_{20}$ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule $-(CH_2)_a-$ , dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

Le ou les polycondensat(s) sont choisis parmi les polycondensats comprenant au moins un motif siliconé.

Le moyen de distribution, qui forme une partie du dispositif aérosol, est généralement constitué par une valve de distribution commandée par une tête de distribution, elle même comprenant une buse par laquelle la composition aérosol est vaporisée.

Conformément à l'invention, le débit initial en composition aérosol est mesuré pour une température voisine de 20°C à l'intérieur du dispositif aérosol. En pratique, on place le dispositif aérosol à la température ambiante et on mesure le débit initial en composition aérosol lorsque l'équilibre thermodynamique est atteint.

Le débit initial est la quantité moyenne de produit sortant sur cinquante secondes du dispositif aérosol non préalablement utilisé. Il est exprimé en gramme par seconde.

Le débit initial de la composition aérosol ($D_{CA}$) correspond à la quantité de composition aérosol (jus + propulseur) sortant par unité de temps du dispositif aérosol non préalablement utilisé. Il est exprimé en g/s et est mesuré par la différence entre le poids de l'aérosol avant ($M_0$) et après ($M_1$) 10 secondes de vaporisation:

$$D_{CA} = (M_0 - M_1) / 10.$$

De manière avantageuse, le dispositif aérosol selon invention est approprié pour obtenir un débit initial en composition aérosol supérieur ou égal à 0,6 gramme par seconde et inférieur ou égal à 1,5 gramme par seconde.
Le débit initial en composition aérosol des dispositifs selon l'invention dépend d'une part de la composition, et d'autre part du moyen de distribution, les deux devant être appropriés pour obtenir les caractéristiques recherchées.
Les caractéristiques particulières définies ci-dessus peuvent être obtenues en sélectionnant les moyens de distribution appropriés et/ou en agissant sur la formulation.
Les polycondensats utilisés conformément à l'invention peuvent être solubles dans le milieu cosmétiquement acceptable ou encore former une dispersion dans ce milieu. La dispersion peut comprendre alors au moins 0,05 % de tensioactif permettant la mise en dispersion et le maintien en dispersion du polycondensat.
Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.
La composition conforme à l'invention comprend, avantageusement, en proportion relative en poids par rapport au poids total de la composition, entre 4,8 et 15 % du polycondensat comprenant au moins une séquence polyuréthanne et/ou polyurée, et plus avantageusement encore entre 5 et 12 % de ce polycondensat.
La composition conforme à l'invention comprend, avantageusement, en proportion relative en poids par rapport au poids total de la composition, entre 0,5 et 3 % du polymère fixant dont la température de transition vitreuse est comprise entre 40 et 200 °C, et plus avantageusement encore entre 0,7 et 2 % de ce polymère fixant.
La température de transition vitreuse du polymère fixant (ii) est avantageusement comprise entre 50 °C et 180 °C, et encore plus préférentiellement entre 60 °C et 170 °C.
Parmi les polymère fixants, dont la température de transition vitreuse est comprise entre 40 °C et 200 °C, on peut tout particulièrement citer le polyvinylca-prolactame commercialisé par la Société BASF, sous l'appellation LUVISKOL PLUS.
Conformément à l'invention, le solvant organique est notamment choisi dans le groupe comprenant les alcools en $C_1$ à $C_4$ tels que l'éthanol ou l'isopropanol, l'acétone, la méthyléthylcétone, l'acétate de méthyle, l'acétate de butyle, l'acétate d'éthyle, le diméthoxyéthane, le diéthoxyéthane et leurs mélanges. De manière préférentielle, on utilise l'éthanol.
La proportion relative en poids, par rapport au poids total de la composition, en gaz propulseur dans la composition est avantageusement comprise entre 15 et 85 %, et plus avantageusement encore entre 25 et 60 %, et encore plus avantageusement entre 30 et 55 %.
Conformément à l'invention, on utilise, de préférence, comme gaz propulseur, un gaz soluble ou non dans la composition tel que le diméthyl éther, les hydrocarbures fluorés ou non, les gaz liquéfiés usuels ou un mélange de ces gaz propulseurs. De préférence, on utilise le diméthyl éther.
Les compositions conformes à l'invention peuvent par ailleurs contenir des additifs cosmétiques conventionnels choisis notamment parmi les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés autres que ceux de l'invention, les silicones volatiles ou non, notamment les silicones anioniques, les polyols, les protéines et les vitamines.
En particulier, il peut être avantageux d'ajouter à la composition d'autres polyméres fixants tels que des polymères fixants non ioniques, anioniques, cationiques ou amphotères, de température de transition vitreuse inférieure à 40 °C.
L'invention pourra être mieux comprise à l'aide de l'exemple non limitatif ci-après.

Exemple:

On réalise un dispositif conforme à l'invention contenant la composition 1 ci-après.

- Luviset Si Pur commercialisé par BASF 5 %
- Luviskol Plus commercialisé par BASF 1 %
- Eau déminéralisée 14 %
- Ethanol 35 %
- Diméthyléther 45%

Le débit initial mesuré à 20°C est de 0,58 gramme par seconde.
On utilise ce dispositif d'une part pour appliquer la composition 1 sur des perruques de cheveux eurochâtains,

et sur lesquels ont a pratiqué un brushing.

Le brushing est particulièrement résistant au vent, dont la force est d'environ 60 km/heure, modélisé par un ventilateur.

On réalise un brushing sur dix personnes. On applique la composition 1 sur leur cheveux. La coiffure tient de manière satisfaisante pendant au moins 4 heures. On coiffe les personnes après 4 heures. Les cheveux se démêlent facilement et sont agréables au toucher.

## Revendications

**1.** Dispositif aérosol comprenant un récipient contenant une composition capillaire formée par un jus et au moins un propulseur ainsi que des moyens de distribution de la composition, **caractérisé par le fait que**:

(1) la composition comprend, dans un milieu cosmétiquement acceptable :

(i) entre 4,5 et 20 % en poids par rapport au poids total de la composition d'au moins un polycondensat comprenant au moins une séquence polyuréthanne et/ou polyurée;
(ii) entre 0,1 et 5 % en poids par rapport au poids total de la composition d'au moins un polymère fixant, différent(s) du ou des polycondensat(s) (i), dont la température de transition vitreuse est comprise entre 40 et 200 °C ;

(2) le dispositif est approprié pour fournir un débit initial en composition aérosol supérieur ou égal à 0,55 gramme par seconde et inférieur ou égal à 2 grammes par seconde,

le polycondensat présentant un motif répétitif de base répondant à la formule (II'):

-X'-P-X'-CO-NH-R-NH-CO- (II')

dans laquelle :

- P est un segment polysiloxanique,
- X' représente O et/ou NH, et
- R est un radical divalent choisi parmi les radicaux alkylène de type aromatique, aliphatique en $C_1$ à $C_{20}$, cycloaliphatique en $C_1$ à $C_{20}$, ces radicaux étant substitués ou non.

**2.** Dispositif selon la revendication 1, **caractérisé par le fait que** le débit initial en composition aérosol est supérieur ou égal à 0,6 gramme par seconde et inférieur ou égal à 1,5 gramme par seconde.

**3.** Dispositif selon l'une quelconque des revendications précédentes, **caractérisé par le fait que** la température de transition vitreuse du polymère fixant (ii) est comprise entre 50 et 180 °C.

**4.** Dispositif selon l'une quelconque des revendications précédentes, **caractérisé par le fait que** la température de transition vitreuse du polymère fixant (ii) est comprise entre 60 et 170 °C.

**5.** Dispositif selon l'une quelconque des revendications précédentes, **caractérisé par le fait qu'**il comprend, en proportion relative en poids par rapport au poids total de la composition, entre 4,8 et 15 % du polycondensat comprenant au moins une séquence polyuréthanne et/ou polyurée, et plus avantageusement encore entre 5 et 12 % de ce polycondensat.

**6.** Dispositif selon l'une quelconque des revendications précédentes, **caractérisé par le fait qu'**il comprend, en proportion relative en poids par rapport au poids total de la composition, entre 0,5 et 3 % du polymère fixant dont la température de transition vitreuse est comprise entre 40 et 200°C, et plus avantageusement encore entre 0,7 et 2 %.

**7.** Dispositif selon l'une quelconque des revendications précédentes, **caractérisé par le fait que** le gaz propulseur est présent à une concentration relative en poids comprise entre 15 et 70 %, de préférence entre 25 et 60 %, et plus avantageusement entre 30 et 50 %.

**8.** Dispositif selon l'une quelconque des revendications précédentes, **caractérisé par le fait que** la composition

contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés autres que ceux de l'invention, les silicones volatiles ou non, notamment les silicones anioniques, les polyols , les protéines et les vitamines.

**9.** Dispositif selon l'une quelconque des revendications précédentes, **caractérisé par le fait que** la composition contient au moins un polymère fixant additionnel choisi dans le groupe comprenant les polymères fixants non ioniques, cationiques, anioniques ou amphotères, de température de transition vitreuse inférieure à 40 °C.

**10.** Procédé capillaire pour la mise en forme ou le maintien de la coiffure, **caractérisé par le fait qu'**il comprend la mise en oeuvre d'un dispositif conforme à l'une quelconque des revendications 1 à 9.

**11.** Utilisation d'un dispositif selon l'une quelconque des revendications 1 à 9 pour la fabrication d'une laque ou d'un spray aérosol.

## Claims

**1.** Aerosol device comprising a container containing a hair composition formed from a fluid and at least one propellant, and also means for dispensing the composition, **characterized in that**:

(1) the composition comprises, in a cosmetically acceptable medium:

(i) between 4.5% and 20% by weight, relative to the total weight of the composition, of at least one polycondensate comprising at least one polyurethane and/or polyurea block;
(ii) between 0.1% and 5% by weight, relative to the total weight of the composition, of at least one fixing polymer other than the polycondensate(s) (i), with a glass transition temperature of between 40 and 200°C;

(2) the device is suitable for supplying an initial flow rate of aerosol composition of greater than or equal to 0.55 gram per second and less than or equal to 2 grams per second, the polycondensate having a base repeating unit corresponding to formula (II'):

$$- X' - P - X' - CO - NH - R - NH - CO - \qquad (II')$$

in which

- P is a polysiloxane segment,
- X' represents O and/or NH, and
- R is a divalent radical chosen from the alkylene radicals of aromatic, $C_1$ to $C_{20}$ aliphatic and $C_1$ to $C_{20}$ cycloaliphatic type, these radicals being substituted or unsubstituted.

**2.** Device according to Claim 1, **characterized in that** the initial flow rate of aerosol composition is greater than or equal to 0.6 gram per second and less than or equal to 1.5 grams per second.

**3.** Device according to either of the preceding claims, **characterized in that** the glass transition temperature of the fixing polymer (ii) is between 50 and 180°C.

**4.** Device according to any one of the preceding claims, **characterized in that** the glass transition temperature of the fixing polymer (ii) is between 60 and 170°C.

**5.** Device according to any one of the preceding claims, **characterized in that** it comprises, as a relative weight proportion, relative to the total weight of the composition, between 4.8% and 15% of the polycondensate comprising at least one polyurethane and/or polyurea block, and even more advantageously between 5% and 12% of this polycondensate.

**6.** Device according to any one of the preceding claims, **characterized in that** it comprises, as a relative weight proportion, relative to the total weight of the composition, between 0.5% and 3% of the fixing polymer with a glass transition temperature of between 40 and 200°C, and even more advantageously between 0.7% and 2%.

**7.** Device according to any one of the preceding claims, **characterized in that** the propellent gas is present at a relative weight concentration of between 15% and 70%, preferably between 25% and 60% and more advantageously between 30% and 50%.

**8.** Device according to any one of the preceding claims, **characterized in that** the composition also contains conventional cosmetic additives chosen from the group comprising fatty substances, thickeners, softeners, antifoams, moisturizers, antiperspirants, basifying agents, colorants, pigments, fragrances, preserving agents, surfactants, hydrocarbon-based polymers other than those of the invention, volatile or nonvolatile silicones, especially anionic silicones, polyols, proteins and vitamins.

**9.** Device according to any one of the preceding claims, **characterized in that** the composition contains at least one additional fixing polymer chosen from the group comprising nonionic, cationic, anionic or amphoteric fixing polymers with a glass transition temperature of less than 40°C.

**10.** Haircare process for shaping or holding the hairstyle, **characterized in that** it comprises the use of a device in accordance with any one of Claims 1 to 9.

**11.** Use of a device according to any one of Claims 1 to 9, for the manufacture of an aerosol lacquer or spray.

**Patentansprüche**

**1.** Aerosolvorrichtung, die einen Behälter, der eine Zusammensetzung für die Haarbehandlung enthält, die aus einer Flüssigkeit und mindestens einem Treibmittel gebildet wird, sowie Einrichtungen für die Verteilung der Zusammensetzung umfasst, **dadurch gekennzeichnet, dass**

(1) die Zusammensetzung in einem kosmetisch akzeptablen Medium enthält:

(i) 4,5 bis 20 Gew.-% mindestens eines Polykondensats, das mindestens eine Polyurethansequenz und/oder Polyharnstoffsequenz aufweist, bezogen auf das Gesamtgewicht der Zusammensetzung;
(ii) 0,1 bis 5 Gew.-% mindestens eines fixierenden Polymers, das (die) von dem oder den Polykondensat(en) (i) verschieden ist (sind) und dessen Glasübergangstemperatur im Bereich von 40 bis 200 °C liegt, bezogen auf das Gesamtgewicht der Zusammensetzung;

(2) die Vorrichtung geeignet ist, einen anfänglichen Durchsatz der Aerosolzusammensetzung größer oder gleich 0,55 Gramm pro Sekunde und kleiner oder gleich 2 Gramm pro Sekunde zu liefern,

wobei das Polykondensat eine wiederkehrende Grundeinheit der Formel (II') aufweist

**-X'- P- X'- CO- NH- R- NH- CO-** (II')

worin bedeuten:

- P ein Polysiloxansegment,
- X' O und/oder NH, und
- R eine zweiwertige Gruppe, die ausgewählt ist unter den Alkylengruppen vom Typ aromatisch, aliphatisch mit $C_1$ bis $C_{20}$, cycloaliphatisch mit $C_1$ bis $C_{20}$, wobei diese Gruppen substituiert oder unsubstituiert sind.

**2.** Vorrichtung nach Anspruch 1, **dadurch gekennzeichnet, dass** der anfängliche Durchsatz der Aerosolzusammensetzung größer oder gleich 0,6 Gramm pro Sekunde und kleiner oder gleich 1,5 Gramm pro Sekunde ist.

**3.** Vorrichtung nach einem der vorhergehenden Ansprüche, **dadurch gekennzeichnet, dass** die Glasübergangstemperatur des fixierenden Polymers (ii) im Bereich von 50 bis 180 °C liegt.

4. Vorrichtung nach einem der vorhergehenden Ansprüche, **dadurch gekennzeichnet, dass** die Glasübergangstemperatur des fixierenden Polymers (ii) im Bereich von 60 bis 170 °C liegt.

5. Vorrichtung nach einem der vorhergehenden Ansprüche, **dadurch gekennzeichnet, dass** sie 4,8 bis 15 % Polykondensat, das mindestens eine Polyurethansequenz und/oder Polyharnstoffsequenz aufweist, bezogen auf das Gesamtgewicht der Zusammensetzung, und noch vorteilhafter 5 bis 12 % Polykondensat enthält.

6. Vorrichtung nach einem der vorhergehenden Ansprüche, **dadurch gekennzeichnet, dass** sie 0,5 bis 3 % fixierendes Polymer, dessen Glasübergangstemperatur im Bereich von 40 bis 200 °C liegt, bezogen auf das Gesamtgewicht der Zusammensetzung, und noch vorteilhafter 0,7 bis 2 % fixierendes Polymer enthält.

7. Zusammensetzung nach einem der vorhergehenden Ansprüche, **dadurch gekennzeichnet, dass** das Treibmittel in einer relativen Konzentration von 15 bis 70 Gew.-%, vorzugsweise 25 bis 60 Gew.-% und noch vorteilhafter 30 bis 50 Gew.-% enthalten ist.

8. Zusammensetzung nach einem der vorhergehenden Ansprüche, **dadurch gekennzeichnet, dass** die Zusammensetzung ferner herkömmliche kosmetische Zusatzstoffe enthält, die unter den Fettsubstanzen, Verdickungsmitteln, beruhigenden Stoffen, Schaumverhütungsmitteln, Hydratisierungsmitteln, Antiperspirantien, Alkalisierungsmitteln, Farbmitteln, Pigmenten, Parfums, Konservierungsmitteln, grenzflächenaktiven Stoffen, Polymeren auf Kohlenwasserstoffbasis, die von den erfindungsgemäßen Polymeren verschieden sind, flüchtigen oder nichtflüchtigen Siliconen und insbesondere anionischen Siliconen, Polyolen, Proteinen und Vitaminen ausgewählt sind.

9. Vorrichtung nach einem der vorhergehenden Ansprüche, **dadurch gekennzeichnet, dass** die Zusammensetzung mindestens ein ergänzendes fixierendes Polymer enthält, das unter den nichtionischen, kationischen, anionischen oder amphoteren fixierenden Polymeren mit einer Glasübergangstemperatur unter 40 °C ausgewählt ist.

10. Haarkosmetisches Verfahren für die Formgebung oder Festigung der Frisur, **dadurch gekennzeichnet, dass** es die Verwendung einer Vorrichtung nach einem der Ansprüche 1 bis 9 umfasst.

11. Verwendung einer Vorrichtung nach einem der Ansprüche 1 bis 9 für die Herstellung eines Lacks oder Aerosolsprays.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 19807908
- EP 845257 A
- EP 848941 A
- EP 0751162 A
- EP 0637600 A
- FR 2743297
- EP 0648485 A
- EP 814764 A
- EP 0656021 A
- WO 9403510 A
- EP 0619111 A